# EUROPEAN PATENT SPECIFICATION

(11) **EP 3 714 911 B1**
(45) Date of publication and mention of the grant of the patent: **15.02.2023**
(21) Application number: 20165889.5
(22) Date of filing: 26.03.2020
(51) Int. Cl.: A61L 27/30, A61L 27/50

(54) **COATING FOR JOINT IMPLANTS**
BESCHICHTUNG FÜR GELENKIMPLANTATE
REVÊTEMENT POUR IMPLANTS DE JOINT

(30) Priority: 29.03.2019 US 201962826603 P
(43) Date of publication of application: 30.09.2020
(73) Proprietor: Picosun Oy, 02150 Espoo (FI)
(72) Inventor: BLOMBERG, Tom, 01260 Vantaa (FI); KOSTAMO, Juhana, 02260 Vantaa (FI); OKSALA, Niku, 33700 Tampere (FI)
(74) Representative: Heinonen & Co

(56) References cited:
- WO-A1-97/16137
- WO-A1-2013/143857
- WO-A2-2013/040461
- CA-A1- 2 671 064
- US-A- 4 687 487
- US-A1- 2006 251 875
- US-A1- 2012 114 936
- US-A1- 2015 118 649
- DATABASE WPI Week 201823 Thomson Scientific, London, GB; AN 2018-17165M XP002800111, & CN 107 723 680 A (UNIV NANCHANG) 23 February 2018 (2018-02-23) -& CN 107 723 680 A (UNIV NANCHANG) 23 February 2018 (2018-02-23)
- LUTING LIU ET AL: "Atomic layer deposition of nano-TiO<sub>2 </sub>thin films with enhanced biocompatibility and antimicrobial activity for orthopedic implants", INTERNATIONAL JOURNAL OF NANOMEDICINE, vol. Volume 12, 1 December 2017 (2017-12-01), pages 8711-8723, XP055724261, DOI: 10.2147/IJN.S148065

## Description

### FIELD OF THE INVENTION

The present invention generally relates to chemical deposition methods for coating joints implants, coated articles and uses.

### BACKGROUND OF THE INVENTION

In biological joints, cartilage tissue covers and protects the ends of bones at the joints and acts as a lubricant preventing the articulating bones from contacting one another while remaining in the area of contact. Joint replacement implants naturally lack this protection.

Hence, a common problem with joint replacement implants is wear, tear and loosening (aseptic or infectious) that ultimately result in a need to replace the joint, i.e. in revision joint replacement. Revision surgery employs excessive costs, impaired patient recovery, socieconomical burden and high risk of complications. The risk of complications is determined by both patient-related and implant-related factors. Overall, the patients of younger age generally tend to exercise higher impact activities, which accounts for excessive implant wear and tear. The same stands for the patients with excessive weight.

A joint implant is commonly composed of a polished bearing having metal surfaces (metal-on-metal implant). Alternatively, the implant can be provided with a plastic spacer between the metal surfaces (metal-on-plastic). Depending on a medical application and the implant type, joint implants can be also configured with ceramic surfaces having metal parts, which are fitted into the bone. With respect to hip joints, the metal-on-metal implants have up to two-fold rate of complications, as compared to ceramic implants, and up to four-fold rate of complications, as compared to conventional metal implants with plastic spacers.

Continuous friction between joint surfaces causes wearing of the implant parts (the interfacing surfaces) and possibly wearing of the plastic spacer, which altogether resulting in bone loss and subsequent loosening of the implant. The mechanism is aseptic, i.e. noninfectious, when the loosening is associated with surface wearing. In such an event, wearing results in alteration of biomechanical loading properties accompanied with release of both plastic- (commonly polyethylene) and metallic (commonly cobalt and titanium) microparticles into surrounding tissue. This results in subsequent activation of immune system, which initiates the removal of foreign particles causing lysis of the bone and soft tissues around the implant (metallosis and osteolysis). Subsequently, this accelerates the implant loosening due to profound biomechanical alterations. Ultimately, ion accumulation in to the body may result in neurological disturbances.

The metal and plastic surfaces also form a favorable environment for bacteria to reside on the implant. This results in formation of antibiotic-resistant biofilms due to either infections during a primary operation (early infections) or infections entered through the bloodstream (later infections). Apart from the implications caused thereby, continuous infections with or without loosening the joint often lead to a necessity of revising the joint.

In this regard, an update in the field of modifying the surfaces of medical implants is still desired. In particular, the challenges associated with reducing wear and tear in joint implants should be addressed.

WO2013040461 discloses implantable bone prostheses formed from elements having articulating surfaces coated with hard and/or abrasion resistant materials which reduce the coefficient of friction, whereby the friction reducing coating is applied to the first and the second articulating surface by PVD or CVD.

### SUMMARY

An objective of the present invention is to solve or to at least alleviate each of the problems arising from the limitations and disadvantages of the related art. The objective is achieved by various embodiments of a method for coating joint endoprostheses, coated articles and related uses. Thereby, in one aspect of the invention a method for depositing a coating film on at least a part of a joint endoprosthesis is provided, according to what is defined in the independent claim 1, which is a method for depositing a coating film or films with atomic layer deposition (ALD) on the surfaces of at least two separate elements (11A, 11B) which establish a joint endoprosthesis (10), and have contact surfaces (12A, 12B) configured to face one another to establish an artificial joint and non-contact surfaces (14A, 14B) other than the contact surfaces, said method comprising: on the contact surfaces (12A, 12B) of each said element, ALD-depositing a low-friction coating film configured to reduce friction of the joint, and 10 on the non-contact surfaces (14A, 14B) of each said element, ALD-depositing an adhesion-improving coating film configured to promote adhesion of the endoprosthesis to a surrounding tissue.

In embodiment, the method is provided for depositing a coating film on at least a part of a joint endoprosthesis composed of at least two separate elements having contact surfaces configured to face one another to establish an artificial joint, with atomic layer deposition (ALD) such, that the contact surface of each said element is deposited with the coating film configured to reduce friction of the joint.

In embodiment, the method comprises depositing the surfaces of the joint endoprosthesis other, than the contact surfaces, with the coating film that promotes adhesion and growth of bone tissue around said endoprosthesis.

In embodiment, the method comprises depositing the coating film that comprises a metal-containing compound.

In embodiment, the method comprises depositing the coating film, in which the metal-containing compound is a titanium (Ti) compound selected from titanium nitride (TiN) and titanium oxynitride (TiOₓN_{y}).

In another aspect of the invention, a joint endoprosthesis is provided, according to what is defined in the independent claim 5, which is a joint endoprosthesis (10) established with at least two separate elements (11A, 11B), the surfaces of each said element (11A, 11B) comprise the coating film or films deposited with atomic layer deposition (ALD), wherein each said element (11A, 11B) has contact surfaces (12A, 12B) configured to face one another to establish an artificial joint and non-contact surfaces (14A, 14B) other than the contact surfaces, wherein the contact surfaces (12A, 12B) of each said element comprise a low-friction coating film configured to reduce friction of the joint, and wherein the non-contact surfaces (14A, 14B) of each said element comprise an adhesion-improving coating film configured to promote adhesion of the endoprosthesis to a surrounding tissue.

In embodiment, the joint endoprosthesis is established with at least two separate elements having contact surfaces configured to face one another such, as to establish an artificial joint, wherein the contact surface of each said element comprises a coating film deposited with atomic layer deposition (ALD), said coating film being configured to reduce friction of the joint.

In embodiment, the surfaces other, than the contact surfaces are deposited with a coating film that promotes adhesion and growth of bone tissue around said endoprosthesis.

In embodiment, the joint endoprosthesis is configured as a ball-joint endoprosthesis.

In embodiment, the joint endoprosthesis is configured to establish a point of contact between at least two articulating bones.

In embodiment, each element in the joint endoprosthesis is independently composed of any one of metal, polymer or ceramics.

In embodiment, the coating film comprises a metal-containing compound. In embodiment, said metal-containing compound is a titanium (Ti) compound selected from titanium nitride (TiN) and titanium oxynitride (TiOₓN_{y}).

In another aspect of the invention, a use of a titanium nitride based compound in a friction reducing coating film for a joint endoprosthesis is provided, according to what is defined in the independent claim 12, which is the use of a titanium nitride based compound and of titanium oxide in producing a coating film or films with atomic layer deposition (ALD) on the surfaces of at least two separate elements (11A, 11B) which establish a joint endoprosthesis (10) and have contact surfaces (12A, 12B) configured to face one another to establish an artificial joint and non-contact surfaces (14A, 14B) other than the contact surfaces, wherein the contact surfaces (12A, 12B) of each said element comprise a low-friction coating film deposited with the titanium nitride based compound and configured to reduce friction of the joint and, and wherein the non-contact surfaces (14A, 14B) of each said element comprise an adhesion-improving coating film deposited with titanium oxide and configured to promote adhesion of the endoprosthesis to a surrounding tissue.

Said use is provided in the joint endoprosthesis composed of at least two separate elements having contact surfaces configured to face one another to establish an artificial joint, said coating film being deposited with atomic layer deposition.

In embodiment, the titanium nitride based compound is selected from titanium nitride and titanium oxynitride.

Without limiting the scope and interpretation of the patent claims, certain technical effects of one or more of the example embodiments disclose herein are listed in the following.

The invention offers a simple and cost-effective method to produce a wear-resistant and friction-preventive coating on joint endoprostheses. E.g. titaniumcompound containing coatings prevent harmful microorganisms from residing on the coated surfaces, thus markedly reducing the rate of infections and other postoperative complications.

The invention provides means to improve metallic, ceramic or plastic surfaces to counteract wear and tear and to reduce ability of bacteria to adhere on the surfaces. The latter makes the invention particularly beneficial in preventing aseptic and/or infectious loosening of joint implants and, ultimately, in preventing accumulation of ions released from the joint surfaces, which would otherwise result in systemic medical conditions.

Additionally, the invention provides a versatile tool for modulating surface properties of the implant such, that certain parts of the implant can be deposited with a coating that promotes interfacing of the implant to the surrounding tissue, such as bone.

In the present disclosure, materials with a layer thickness below 1 micrometer (µm) are referred to as "thin films".

In present disclosure, the terms "implant" and "endoprosthesis" are used interchangeably.

The expression "a number of" refers herein to any positive integer starting from one (1), e.g. to one, two, or three; whereas the expression "a plurality of" refers herein to any positive integer starting from two (2), e.g. to two, three, or four.

The terms "first" and "second" are not intended to denote any order, quantity, or importance, but rather are used to merely distinguish one element from another.

### BRIEF DESCRIPTION OF THE DRAWINGS

The invention will now be described, by way of example only, with reference to the accompanying drawings, in which:
Fig. 1 schematically illustrates a joint endoprosthesis, according to an embodiment;
Fig. 2 schematically illustrates an endoprosthesis, according to an exemplary embodiment, for a hip joint.

### DETAILED DESCRIPTION

The invention concerns provision of a low-friction, wear-resistant deposition coatings on endoprostheses or surgical implants, preferably, configured as joint endoprostheses or replacement joints (artificial joints), for example. Additionally, the invention allows for modulating implant coatings in a manner to promote the implant's growth onto the bone tissue.

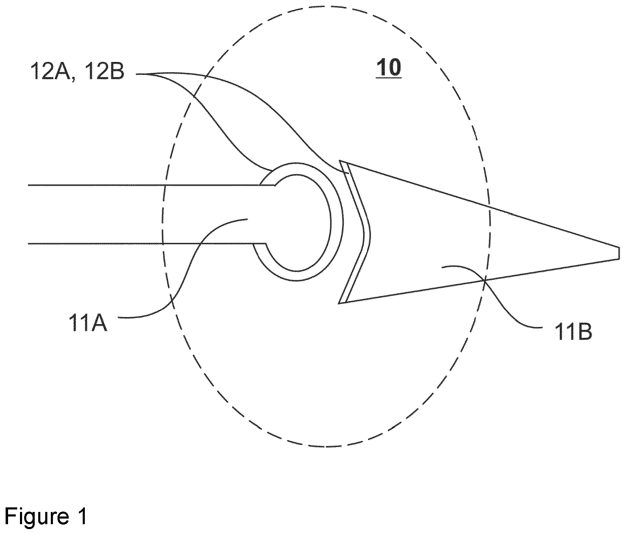

Fig. 1 schematically illustrates a joint endoprosthesis 10, hereafter, a joint 10 (outlined in a circle). The endoprosthesis has a body composed of at least two distinct, separate elements 11A and 11B. The elements 11A, 11B face one another in a manner to establish an artificial joint.

The implant body and/or each element 11A, 11B provided therein is independently composed of any one of: metal or metal alloy, polymer, or ceramics. Mentioned materials constitute the implant base material.

The elements 11A, 11B forming the joint 10 face each other by virtue of predetermined surfaces, designated as the contact surfaces. Each element 11A, 11B has a contact surface 12A, 12B, accordingly. The elements 11A, 11B face one another by virtue of said contact surfaces 12A, 12B.

In the method, a coating film is deposited on at least a part of the joint endoprosthesis 10 described hereinabove. The coating film comprising at least one coating layer (deposition layer) is deposited by Atomic Layer Deposition (ALD). Coating substances for depositing the coating film on the contact surfaces 12A, 12B are selected such, as to render these surfaces more slidable and to reduce friction between the elements 11A, 11B, accordingly. The coating film generated on the surfaces 12A, 12B acts as a lubricant that ensures coordinated optimal operation between articulating / moving parts in the joint. The coating film can be deposited on the surfaces 12A, 12B using the same chemicals. Alternatively, different coating chemicals can be selected separately for each contact surface 12A, 12B to produce on each individual surface 12A, 12B a film different in terms of composition, but still preserving necessary friction-reducing functionality.

According to the invention, Atomic Layer Deposition (ALD) technology is used to produce film coating(s). ALD technology is particularly suitable for such purpose, as it enables producing thin films (few nanometer thick) on wide variety of surfaces, including that used in manufacturing of implants. Additionally, ALD methods allow for generating coating(s) from wide variety of chemicals, including the ones with lubricating / friction reduction properties.

Furthermore, by virtue of its conformal nature, the ALD process allows for filling nanocracks or voids on the contact surfaces 12A, 12B, thus sealing the surface(s) and improving their resistance to wear.

In some instances, the coating film comprises at least one metal-containing compound.

The basics of an ALD growth mechanism are known to a skilled person. ALD is a special chemical deposition method based on the sequential introduction of at least two reactive precursor species to at least one substrate. It is to be understood, however, that one of these reactive precursors can be substituted by energy when using, for example, photon-enhanced ALD or plasma-assisted ALD, for example PEALD, leading to single precursor ALD processes. For example, deposition of a pure element, such as metal, requires only one precursor. Binary compounds, such as oxides can be created with one precursor chemical when the precursor chemical contains both of the elements of the binary material to be deposited. Thin films grown by ALD are dense, pinhole free and have uniform thickness.

The at least one substrate is typically exposed to temporally separated precursor pulses in a reaction vessel to deposit material on the substrate surfaces by sequential self-saturating surface reactions. In the context of this application, the term ALD comprises all applicable ALD based techniques and any equivalent or closely related technologies, such as, for example the following ALD sub-types: MLD (Molecular Layer Deposition), plasma-assisted ALD, for example PEALD (Plasma Enhanced Atomic Layer Deposition) and photon-enhanced Atomic Layer Deposition (known also as photo-ALD or flash enhanced ALD). It should be noted that with PEALD and photon-enhanced ALD, the additive treatment can be limited to the surfaces visible to the radiation source.

ALD is based on alternating self-saturative surface reactions, wherein different reactants (precursors) provided as chemical compounds or elements in a nonreactive (inert) gaseous carrier are sequentially pulsed into a reaction space accommodating a substrate. Deposition of a reactant is followed by purging the substrate by inert gas. Conventional ALD deposition cycle proceeds in two halfreactions (pulse A - purge A; pulse B - purge B), whereby a layer of material is formed in a self-limiting (self-saturating) manner, typically being 0,05 - 0,2 nm thick. Typical substrate exposure time for each precursor ranges within 0,01 - 1 seconds.

Pulse A comprises a first precursor in a gaseous phase (first precursor vapor) and pulse B comprises a second precursor in a gaseous phase (second precursor vapor). Inactive gas and a vacuum pump are typically used for purging gaseous reaction by-products and the residual reactant molecules from the reaction space during purge A and purge B. A deposition sequence comprises at least one deposition cycle. Deposition cycles are repeated until the deposition sequence has produced a thin film or coating of desired thickness. Deposition cycles can also be either simpler or more complex. For example, the cycles can include three or more reactant vapor pulses separated by purging steps, or certain purge steps can be omitted. On the other hand, photo-enhanced ALD has a variety of options, such as only one active precursor, with various options for purging. All these deposition cycles form a timed deposition sequence that is controlled by a logic unit or a microprocessor.

By way of example, to provide an ALD coating film, in at least one ALD deposition cycle, at least two precursors should be sequentially pulsed into the reaction space (with purges of inert gas in between). In ALD, each coating material (precursor) must be delivered into the reaction space one at a time. Therefore, ALD coating on the surfaces 12A, 12B (elements 11A, 11B) is implemented by sequentially directing first- and second precursors into the reaction space, wherein said precursors are allowed to react with each other to produce a deposition (sub)layer. A coating film of desired thickness (comprising a desired number of deposition (sub)layers) is formed in a number of deposition cycles. The coating film may comprise a number of such deposition (sub)layers, wherein each subsequent (sub)layer is "stacked" on the top of a preceding (sub)layer.

In the method, the exemplary metal-containing compound is a titanium (Ti) compound. The metal-containing compound may comprise titanium, nitrogen and/or oxygen. In some exemplary configurations, the metal-containing compound is titanium nitride (TiN) or titanium oxynitride (TiOₓN_{y}). In some instances, the titanium compound is titanium oxide, including, but not limited with titanium (IV) dioxide (TiO₂), titanium (II) oxide (TiO), and titanium (III) oxide (Ti₂O₃).

In some instances, the method provides for coating with TiN/TiOₓN_{y} alloy.

In the method, deposition coating is preferably applied solely (not according to the claimed invention) over the contact surfaces 12A, 12B of the elements 11A, 11B that establish the artificial joint. In some instances, an entire surface of the implant can be coated (as described further below). Coating is naturally applied by placing the implant body into a reaction chamber of a chemical deposition reactor, preferably, the ALD reactor, and conducting a chemical reaction or reactions

TiN coating has been used as a low-friction, wear resistant coating in cutting and drilling tools. TiN coating film applied on an item by the ALD process is highly conformal and has very low surface roughness. In exemplary configurations, TiN has been utilized to produce a coating film on the contact surfaces 12A, 12B of the implant joints aiming at reducing friction and wear of the joint.

TiN coating can be deposited in an exemplary TiX_{y} + NH₃ process, where X is a halide element. In addition to ammonia (NH₃), also other nitrogen-containing precursors can be used, such as hydrazine (N₂H₄), N*, or NH₃*, where marking (*) means that the precursor molecule is an ion or a radical (an excited state molecule). Instead of titanium halides, organometallic- or metalorganic Tiprecursors can be used.

In another exemplary configuration, titanium oxynitride TiON has been used as the ALD coating material, to produce the coating film. Addition of oxygen into the titanium nitride (TiN) process leads to formation of TiON films, whose surface irregularity (roughness) is typically even lower, in a nanoscale, than that of the pure TiN film. Deposition TiON films can be implemented using same basic chemistry, but by adding an oxygen-containing precursor into the process.

Non-limiting examples of the process sequences are presented herein below.

A reaction of TiX_{y} + NH₃ + H₂O, where precursor can be pulsed in any order and pulsing ratios is illustrated with an exemplary equation (1).

(1) TiX_{y} + N* + O₂ or TiX_{y} + N* + O₃ or TiX_{y} + N* + O*

Similarly to a TiN process, the titanium precursor can also be an organometallic compound or a metalorganic compound.

A particularly useful process for depositing TiON is the process utilizing dry- or humid air as a nitrogen- and oxygen containing precursor. The process is illustrated by an exemplary equation (2):

(2) TiX_{y} + dry air or TiX_{y} + humid air or TiX_{y} + air plasma

The titanium precursor can be an organometallic compound or a metalorganic compound.

In addition to above mentioned titanium nitride compounds, wear-resistant, optionally low-friction, coating films can be established from metal carbide-, nitrideand/or carbonitride compounds, wherein the metal is any one of titanium, aluminium, vanadium, tantalum, boron, tungsten, molybdenum and niobium.

Said low-friction, wear-resistant coating films can thus be established independently from any one of the following compounds: titanium carbide (TiC), titanium aluminium nitride (TiAlN), titanium diboride (TiB₂), vanadium nitride (VN), tantalum nitride (Ta₃N₅), tantalum carbonitride (TaNC), boron nitride (BN), boron carbide (BC), tungsten (W), tungsten carbide (WC), tungsten nitride (WN), tungsten carbonitride (WNₓC_{y}, e.g. WNC), molybdenum nitride (MoN), niobium nitride (NbN).

Hence, the method allows for depositing other, than titanium-containing, substances to produce coating films with enhanced functionalities. For example, the coating films deposited on the contact surfaces 12A, 12B of the elements 11A, 11B can differ in terms of at least coating composition and/or a number of deposition (sub)layers to further modulate the properties of the joint 10. Still, the elements 11A, 11B can be provided with identical coating.

Traditionally, replacement joints are established on porous surfaces or high-gloss surfaces with or without bone cement (cemented prostheses and cementless prostheses, accordingly), or having a combination of these properties. The method disclosed hereby offers a versatile tool to modulate the surface properties of the implant and/or the parts of said implant such, that certain parts of said implant (e.g. contact surfaces 12A, 12B) can be deposited with a low-friction coating, whereas the other parts of the implant can be deposited with a coating that promotes the bone tissue to grow onto / around the implant and to adhere to it over time.

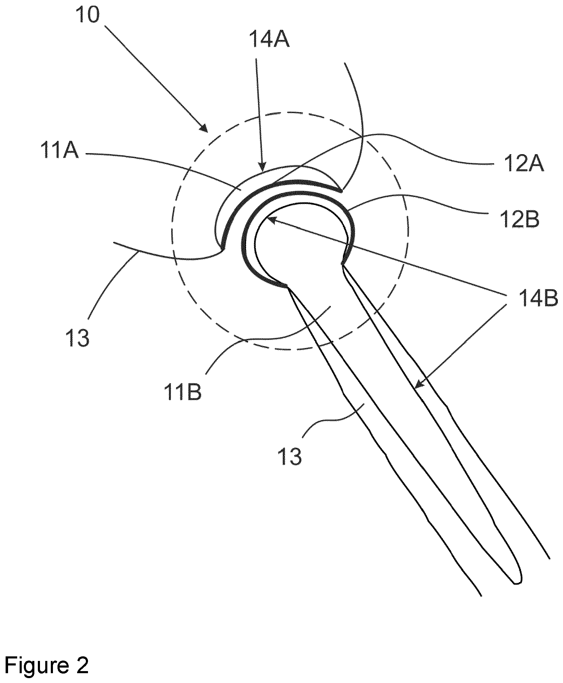

Such configuration is schematically illustrated by Fig. 2.

Fig. 2 is a schematic representation of a joint endoprosthesis 10 having the elements 11A, 11B facing one another. Each element 11A and 11B is incorporated into a bone 13. In the example shown on Fig. 2, the element 11A is provided with a concave surface 12A, forming a contact surface with another part of the implant (11B), and with a convex surface 14A essentially opposite the contact surface 12A and interfacing to the bone 13. The element 11B is, in turn, configured as an elongated body (e.g. a bolt-type implant) having a head with a convex surface 12B (forming the contact surface with the contact surface 12A of the opposite element 11A) and a surface 14B provided as a surface other than the contact surface 12B. The body of the element 11B is surrounded with bone tissue 13 except the part of said element forming the interface with the counterpart 11A. In the present example, the parts forming the interface at the artificial joint are the contact surfaces 12A and 12B and optionally the implant base material laid just under the mentioned surfaces.

We note that the configuration shown on Fig. 2 is merely exemplary; therefore, different configurations of the endoprosthesis 10 can be conceived. The endoprosthesis may thus be constructed as an articulated one-part element or as a solid one-part element.

The elements 11A, 11B can be coated such, as to establish a low-friction coating on the contact surfaces 12A, 12B and to further establish a coating that promotes adhesion of the implant to a surrounding tissue (e.g. bone) on the rest of the implant surface 14A, 14B. The method disclosed hereby allows for applying different coatings on different parts of the joint implant in highly versatile manner.

The coating film deposited on the surfaces other than the contact surfaces 12A, 12B (viz. to the surfaces 14A, 14B) facilitates establishing an interface between the implant and bone and promotes adhesion and growth of bone tissue around the endoprosthesis and parts thereof.

A non-limiting example for establishing an adhesion-improving coating film on the surfaces 14A, 14B is establishing said film with a metal oxide, such as titanium (di)oxide (TiO₂).

A combination can be conceived, including the coating film deposited with a titanium nitride compound, according to the embodiments (e.g. TiN, TiOₓN_{y}), for the contact surfaces 12A, 12B and the coating film deposited with a titanium compound, such as titanium (di)oxide (TiO₂), for the non-contact surfaces 14A, 14B.

Certain compounds, such as titanium nitride based compounds, require only slight modification to attains variations in functionality regarding a predetermined application.

The invention further pertains to provision of a joint endoprosthesis 10 established with at least two separate elements 11A, 11B and comprising an atomic layer deposition (ALD) coating layer deposited over at least a part of each said element.

Figs. 1 and 2 show the endoprosthesis 10 to establish an artificial joint, such as a ball-type joint, for example (outlined in a circle). In such an event, the endoprosthesis comprises at least two separate parts configured as elements 11A, 11B having contact surfaces 12A, 12B configured to face one another, whereby the artificial joint is established. In the ball-type joint, the contact (interfacing) ends (at the elements 11A, 11B) are configured as concave and convex surfaces 12A, 12B facing one another. Overall, any other configuration is possible, as far as a point of contact between at least two articulating bones 13 is established (Fig. 2).

The surfaces of each said element 11A, 11B comprise the coating film or films deposited with atomic layer deposition (ALD).

The joint endoprosthesis 10 is preferably configured to establish a point of contact between at least two articulating bones 13. The coating film at the ends 12A, 12B is configured to serve as a low-friction, wear-resistant coating. The coating film at the portions other than the contact surfaces 12A, 12B is configured as a coating film that promotes adhesion and growth of bone tissue around the endoprosthesis.

The joint endoprosthesis 10 is particularly beneficial for use in replacement surgery. By way of example and not limitation, the endoprosthesis 10 can be provided as an artificial joint for any one of hip, knee, shoulder, elbow and ankle, or as an interphalangeal joint. The joint 10 structure can be configured as a joint implant with fixed- or mobile (e.g. rotating) bearing components.

The endoprosthesis 10 is primarily designed for use in human patients. Upon appropriate modification, the endoprosthesis can be configured for use in nonhuman animals, such as pets and companion animals, for example (dogs, cats, etc.).

The invention further pertains to use of titanium compounds in producing deposition coatings on endoprostheses, in particular, joint endoprostheses. The titanium compounds can be any compound discussed hereinabove, such as the once comprising nitrogen- and/or oxygen. In some instances, said titanium compound is titanium nitride or titanium oxynitride.

The methods and devices discussed hereby is/are applicable to any other type of implants, such as a splint, a bolt, a stent and/or a dental implant.

## Claims

1. A method for depositing a coating film or films with atomic layer deposition (ALD) on the surfaces of at least two separate elements (11A, 11B) which establish a joint endoprosthesis (10), and have contact surfaces (12A, 12B) configured to face one another to establish an artificial joint and non-contact surfaces (14A, 14B) other than the contact surfaces, said method comprising:
on the contact surfaces (12A, 12B) of each said element, ALD-depositing a low-friction coating film configured to reduce friction of the joint, and
on the non-contact surfaces (14A, 14B) of each said element, ALD-depositing an adhesion-improving coating film configured to promote adhesion of the endoprosthesis to a surrounding tissue.

2. The method of claim 1, comprising depositing the non-contact surfaces (14A, 14B) of the joint endoprosthesis with the coating film configured to establish an interface between the endoprosthesis and bone and to promote adhesion and growth of bone tissue around said endoprosthesis.

3. The method of any one of claims 1 and 2, wherein the coating film comprises a metal-containing compound.

4. The method of any preceding claim, wherein the contact surfaces (12A, 12B) are deposited with a titanium nitride based compound selected from titanium nitride (TiN) and titanium oxynitride (TiOₓN_{y}), and wherein the non-contact surfaces (14A, 14B) are deposited with titanium oxide.

5. A joint endoprosthesis (10) established with at least two separate elements (11A, 11B), the surfaces of each said element (11A, 11B) comprise the coating film or films deposited with atomic layer deposition (ALD), wherein each said element (11A, 11B) has contact surfaces (12A, 12B) configured to face one another to establish an artificial joint and non-contact surfaces (14A, 14B) other than the contact surfaces, wherein the contact surfaces (12A, 12B) of each said element comprise a low-friction coating film configured to reduce friction of the joint, and wherein the non-contact surfaces (14A, 14B) of each said element comprise an adhesion-improving coating film configured to promote adhesion of the endoprosthesis to a surrounding tissue.

6. The joint endoprosthesis (10) of claim 5, in which the non-contact surfaces (14A, 14B), are deposited with an adhesion-improving coating film configured to establish an interface between the endoprosthesis and bone and to promote adhesion and growth of bone tissue around said endoprosthesis.

7. The joint endoprosthesis (10) of any one of claims 5 and 6, configured as a ball-joint endoprosthesis.

8. The joint endoprosthesis (10) of any one of claims 5-7, configured to establish a point of contact between at least two articulating bones (13).

9. The joint endoprosthesis (10) of any one of claims 5-8, wherein each said element (11A, 11B) is independently composed of any one of metal, polymer or ceramics.

10. The joint endoprosthesis (10) of any one of claims 5-9, wherein the coating film comprises a metal-containing compound

11. The joint endoprosthesis (10) of any one of claims 5-10, wherein the contact surfaces (12A, 12B) are deposited with a titanium nitride based compound selected from titanium nitride (TiN) and titanium oxynitride (TiOₓN_{y}), and wherein the non-contact surfaces (14A, 14B) are deposited with titanium oxide.

12. Use of a titanium nitride based compound and of titanium oxide in producing a coating film or films with atomic layer deposition (ALD) on the surfaces of at least two separate elements (11A, 11B) which establish a joint endoprosthesis (10) and have contact surfaces (12A, 12B) configured to face one another to establish an artificial joint and non-contact surfaces (14A, 14B) other than the contact surfaces, wherein the contact surfaces (12A, 12B) of each said element comprise a low-friction coating film deposited with the titanium nitride based compound and configured to reduce friction of the joint and, and wherein the non-contact surfaces (14A, 14B) of each said element comprise an adhesion-improving coating film deposited with titanium oxide and configured to promote adhesion of the endoprosthesis to a surrounding tissue.

13. Use of claim 12, wherein the titanium nitride based compound is selected from titanium nitride and titanium oxynitride.

## Patentansprüche

1. Verfahren zum Abscheiden eines Beschichtungsfilms oder Films mittels Atomlagenabscheidung (ALD) auf den Oberflächen von zumindest zwei separaten Elementen (11A, 11B), die eine Gelenkendoprothese (10) bilden und Kontaktflächen (12A, 12B), die dazu ausgelegt sind, zueinander zu zeigen, um ein künstliches Gelenk zu bilden, und neben den Kontaktflächen Nicht-Kontaktflächen (14A, 14B) aufweisen, wobei das Verfahren Folgendes umfasst:
ALD-Abscheidung eines Beschichtungsfilms mit geringer Reibung, der dazu ausgelegt ist, die Reibung des Gelenks zu reduzieren, auf den Kontaktflächen (12A, 12B), und
ALD-Abscheiden eines haftverbessernden Beschichtungsfilms, der dazu ausgelegt ist, die Anhaftung der Endoprothese an einem umgebenden Gewebe zu fördern, auf den Nicht-Kontaktflächen (14A, 14B).

2. Verfahren nach Anspruch 1, umfassend das Abscheiden des Beschichtungsfilms, der zum Bilden einer Grenzfläche zwischen der Endoprothese und Knochen ausgelegt ist, auf den Nicht-Kontaktflächen (14A, 14B) der Gelenkendoprothese, um die Anhaftung und das Wachstum von Knochengewebe um die Endoprothese zu fördern.

3. Verfahren nach einem der Ansprüche 1 und 2, wobei der Beschichtungsfilm eine metallhaltige Verbindung umfasst.

4. Verfahren nach einem der vorhergehenden Ansprüche, wobei auf den Kontaktflächen (12A, 12B) eine Verbindung auf Titannitridbasis, die aus Titannitrid (TiN) und Titanoxinitrid (TiOₓN_{y}) ausgewählt ist, abgeschieden wird, und wobei auf den Nicht-Kontaktflächen (14A, 14B) Titanoxid abgeschieden wird.

5. Gelenkendoprothese (10), die mit mindestens zwei separaten Elementen (11A, 11B) gebildet ist, wobei die Oberflächen jedes der Elemente (11A, 11B) den Beschichtungsfilm oder die -filme, die mittels Atomlagenabscheidung (ALD) abgeschieden werden, umfassen, wobei jedes der Elemente (11A, 11B) Kontaktflächen (12A, 12B), die dazu ausgelegt sind, zueinander zu zeigen, um ein künstliches Gelenk zu bilden, und neben den Kontaktflächen Nicht-Kontaktflächen (14A, 14B) aufweist, wobei die Kontaktflächen (12A, 12B) jedes der Elemente einen Beschichtungsfilm mit geringer Reibung, der dazu ausgelegt ist, die Reibung des Gelenks zu reduzieren, umfasst, und wobei die Nicht-Kontaktflächen (14A, 14B) jedes der Elemente einen haftverbessernden Beschichtungsfilm, der dazu ausgelegt ist, die Anhaftung der Endoprothese an ein umgebendes Gewebe zu fördern, umfasst.

6. Gelenkendoprothese (10) nach Anspruch 5, wobei auf den Nicht-Kontaktflächen (14A, 14B) ein haftungsverbessernder Beschichtungsfilm, der dazu ausgelegt ist, eine Grenzfläche zwischen der Endoprothese und Knochen zu bilden, abgeschieden ist, um die Anhaftung und das Wachstum von Knochengewebe um die Endoprothese zu fördern.

7. Gelenkendoprothese (10) nach einem der Ansprüche 5 und 6, die als Kugelgelenkendoprothese ausgelegt ist.

8. Gelenkendoprothese (10) nach einem der Ansprüche 5 bis 7, die dazu ausgelegt ist, einen Kontaktpunkt zwischen zumindest zwei Gelenkknochen (13) zu bilden.

9. Gelenkendoprothese (10) nach einem der Ansprüche 5 bis 8, wobei jedes der Elemente (11A, 11B) unabhängig aus einem Metall, Polymer oder Keramik besteht.

10. Gelenkendoprothese (10) nach einem der Ansprüche 5 bis 9, wobei der Beschichtungsfilm eine metallhaltige Verbindung umfasst.

11. Gelenkendoprothese (10) nach einem der Ansprüche 5 bis 10, wobei auf den Kontaktflächen (12A, 12B) eine Verbindung auf Titannitridbasis, die aus Titannitrid (TiN) und Titanoxinitrid (TiOₓN_{y}) ausgewählt ist, abgeschieden ist, und wobei auf den Nicht-Kontaktflächen (14A, 14B) Titanoxid abgeschieden ist.

12. Benutzung einer Verbindung auf Titannitridbasis und von Titanoxid zum Herstellen eines Beschichtungsfilms oder -filmen mittels Atomlagenabscheidung (ALD) auf den Oberflächen von zumindest zwei separaten Elementen (11A, 11B), die eine Gelenkendoprothese (10) bilden und Kontaktflächen (12A, 12B), die dazu ausgelegt sind, zueinander zu zeigen, um ein künstliches Gelenk zu bilden, und neben den Kontaktflächen Nicht-Kontaktflächen (14A, 14B) aufweisen, wobei die Kontaktflächen (12A, 12B) jedes der Elemente einen Beschichtungsfilm mit geringer Reibung umfassen, der mit der Verbindung auf Titannitridbasis abgeschieden und dazu ausgelegt ist, die Reibung des Gelenks zu reduzieren, und wobei die Nicht-Kontaktflächen (14A, 14B) jedes der Elemente einen haftverbessernden Beschichtungsfilm umfassen, der mit Titanoxid abgeschieden und dazu ausgelegt ist, die Anhaftung der Endoprothese an ein umgebendes Gewebe zu fördern.

13. Benutzung nach Anspruch 12, wobei die Verbindung auf Titannitridbasis aus Titannitrid und Titanoxinitrid ausgewählt ist.

## Revendications

1. Procédé de dépôt d'un ou de films de revêtement avec un dépôt de couche atomique (ALD) sur les surfaces d'au moins deux éléments distincts (11A, 11B) qui établissent une endoprothèse de joint (10) et ont des surfaces de contact (12A, 12B) configurées pour faire face l'une à l'autre pour établir un joint artificiel et des surfaces de non contact (14A, 14B) différentes des surfaces de contact, ledit procédé comprenant :
sur les surfaces de contact (12A, 12B) de chacun desdits éléments, le dépôt par ALD d'un film à faible frottement configuré pour réduire le frottement du joint et
sur les surfaces de non contact (14A, 14B) de chacun desdits éléments, le dépôt par ALD d'un film de revêtement améliorant l'adhésion configuré pour favoriser l'adhésion de l'endoprothèse à un tissu environnant.

2. Procédé selon la revendication 1, comprenant le dépôt des surfaces de non contact (14A, 14B) de l'endoprothèse de joint avec le film de revêtement configuré pour établir une interface entre l'endoprothèse et l'os et favoriser l'adhésion et la croissance du tissu osseux autour de ladite endoprothèse.

3. Procédé selon l'une quelconque des revendications 1 et 2, dans lequel le film de revêtement comprend un composé contenant du métal.

4. Procédé selon l'une quelconque des revendications précédentes, dans lequel on dépose sur les surfaces de contact (12A, 12B) un composé à base de nitrure de titane choisi parmi le nitrure de titane (TiN) et l'oxynitrure de titane (TiOₓN_{y}) et en déposant sur les surfaces de non contact (14A, 14B) de l'oxyde de titane.

5. Endoprothèse de joint (10) établie avec au moins deux éléments distincts (11A, 11B), les surfaces de chacun desdits éléments (11A, 11B) comprennent le film ou les films de revêtement déposés avec un dépôt de couche atomique (ALD), chacun desdits éléments (11A, 11B) ayant des surfaces de contact (12A, 12B) configurées pour faire face l'une à l'autre pour établir un joint artificiel et des surfaces de non contact (14A, 14B) différentes des surfaces de contact, les surfaces de contact (12A, 12B) de chacun desdits éléments comprenant un film de revêtement à faible frottement configuré pour réduire le frottement du joint, et les surfaces de non contact (14A, 14B) de chacun desdits éléments comprenant un film de revêtement améliorant l'adhésion pour favoriser l'adhésion de l'endoprothèse à un tissu environnant.

6. Endoprothèse de joint (10) selon la revendication 5, dans laquelle on dépose sur les surfaces de non contact (14A, 14B) un film de revêtement améliorant l'adhésion configuré pour établir une interface entre l'endoprothèse et l'os et favoriser l'adhésion et la croissance du tissu osseux autour de ladite endoprothèse.

7. Endoprothèse de joint (10) selon l'une quelconque des revendications 5 et 6, configurée comme une endoprothèse à joint en boule.

8. Endoprothèse de joint (10) selon l'une quelconque des revendications 5 à 7, configurée pour établir un point de contact entre au moins deux os d'articulation (13).

9. Endorpothèse de joint (10) selon l'une quelconque des revendications 5 à 8, dans laquelle chacun desdits éléments (11A, 11B) est composé indépendamment de l'un quelconque d'un métal, d'un polymère ou de céramique.

10. Endoprothèse de joint (10) selon l'une quelconque des revendications 5 à 9, dans laquelle le film de revêtement comprend un composé contenant du métal.

11. Endoprothèse de joint (10) selon l'une quelconque des revendications 5 à 10, dans lequel on dépose sur les surfaces de contact (12A, 12B) un composé à base de nitrure de titane choisi parmi le nitrure de titane (TiN) et l'oxynitrure de titane (TiOₓN_{y}) et en déposant sur les surfaces de non contact (14A, 14B) de l'oxyde de titane.

12. Utilisation d'un composé à base de nitrure de titane et d'oxyde de titane dans la production d'un ou de films de revêtement avec dépôt de couche atomique (ALD) sur les surfaces d'au moins deux éléments distincts (11A, 11B) qui établissent une endoprothèse de joint (10) et ont des surfaces de contact (12A, 12B) configurées pour faire face l'une à l'autre pour établir un joint artificiel et des surfaces de non contact (14A, 14B) différentes des surfaces de contact, les surfaces de contact (12A, 12B) de chacun desdits éléments comprenant un film de revêtement à faible frottement déposé avec le composé à base de nitrure de titane et configuré pour réduire le frottement du joint et, et les surfaces de non contact (14A, 14B) de chacun desdits éléments comprenant un film de revêtement améliorant l'adhésion déposé avec l'oxyde de titane et configuré pour favoriser l'adhésion de l'endoprothèse à un tissu environnant.

13. Utilisation selon la revendication 12, dans laquelle le composé à base de nitrure de titane est choisi parmi le nitrure de titane et l'oxynitrure de titane.
